# EUROPEAN PATENT APPLICATION

(11) **EP 1 103 539 A1**
(43) Date of publication of application: **30.05.2001**
(21) Application number: 99670008.4
(22) Date of filing: 18.11.1999
(51) Int. Cl.: C07C 49/417, C07J 63/00

(54) **Process for extraction and purification of friedelin from cork smoker wash solids**

(71) Applicant: Do Nascimento Junior, José Maria Prof., 2685 Portela De Sacavém (PT)
(72) Inventor: Do Nascimento Junior, José Maria, Prof, 2685 Portela De Savacém (PT); Pereira Gaspar Marques, Cristina Maria, Dr, Reboleira, 2700 Amadora (PT)
(74) Representative: Ferreira, Maria Silvina

(57) **Abstract**

This invention concerns a method for the extraction and separation of friedelin from the different components smoker wash solids, by means of simple mechanical, physical and chemical treatments. These treatments consist in the fragmentation of the resinous residue, percolation with organic solvents or mixtures of organic solvents at temperatures between room temperature and the boiling point of solvents or mixtures and percolation with alkaline salt solutions, followed by distillation and drying of the extracts. These methods are characterized by a final friedelin yield, that may vary beteween 1% and 3%, with a putity degree (HPLC) within the range of 87%-101%.

## Description

### Field of the Invention

The black cork bricks' industry is a predominantly Portuguese activity, which currently processes amounts of about 10 000 tons per year. As a consequence of this activity 13,5% of water soluble residue and 1,5% of a residue commonly referred to as resin, or in the anglo-saxon terminology "smoker wash solids" are produced.

Smoker wash solids are considered an inconvenient for black cork brick factories due to the problems it causes on drain systems and, it has been considered so far an undesirable material for which no other use could be found. It is only sometimes used as a waterproof pavement covering.

This invention concerns new industrially applicable processes for the extraction and separation of friedelin from the different components of smoker wash solids. In these methots single mechanical, physical, and chemical treatments are used, which can easily be scaled-up to industrial production.

### Summary of the Invention

This invention concerns a method for the extraction and separation of friedelin from the different components of smoker wash solids, by means of simple mechanical, physical and chemical treatments. These treatments consist of fragmentation of the resinous residue, percolation with organic solvents or mixtures of organic solvents at temperatures between room temperature and the boiling point of solvents or mixtures and percolation with alkaline salt solutions, followed by distillation and drying of the extracts. These methods are characterized by a final friedelin yield, that may vary between 1% and 3% with a purity degree (HPLC) within the range of 87%-101%.

### Background of the Invention

In 1899, Istrati and Ostrogovich (Compt. Rend. 128, 1581, 1899) presented a study on the identification of friedelin (friedelan-3-one) isolated from cork. Drake (Drake, N.L.; Jacobsen, R.P, J. Am. Chem. Soc., vol. 57, 1935, p. 1570-1573) obtained friedelin by the extraction of cork with ethyl acetate and the recrystallisation with dichloromethane and benzene. Corey and Ursprung (Corey, J. and Ursprung, J.J.; J. Am. Chem. Soc., 78, 1956, p. 5041), isolated friedelin for the first time from the smoker wash solids with the purpose of establishing friedelin structure, followed by Stevenson's studies (Stevenson, R.; J. Org. Chem., vol. 26, 1961, p. 2124-2143) on the same subject. In what concerns friedelin, cork smoker wash solids have unique characteristics, due to the fact that they contain this substance in extraordinarily high amounts (1%-5%), in comparison to what is currently found in nature (typical contents of friedelin: about 0,01 %).

The methods described by these authors and the methods currently used to isolate friedelin, though simple in laboratorial terms, are difficult to extrapolate to industrial scale due to the use of large volumes of organic solvents and the use of cromatographic methods requiring a consumption of adsorbents far superior to the amounts of cork smoker wash solids treated and of a difficult and expensive scaling-up.

The unawareness of any important use of friedelin at the time of the establishment of its chemical structure did not cause its developments, in the sense of making the best of this source, which in terms of abundance and availability is exclusively bound to the existence of the black cork bricks industry.

In the meanwhile, the development of chemistry and pharmacology of natural products led the attention to the interesting biologic properties of triterpenes isolated from vegetable extracts. However, these substances also could not be easily exploited due to difficulties in obtaining relevant amounts for industrial use.

This invention concerns alternative methods for the extraction and separation of friedelin from the several components of a cork smoker wash solids by means of simple chemical and easy scaling-up treatments on an industrial production.

### Detailed Description of the Invention

This invention consists in sequential leaching treatments of cork smoker wash solids with different organic solvents or organic solvents and alkaline salt solutions with the purpose of isolating friedelin. Alkaline salt solutions may be aqueous or hydroalcoholic solutions. Extractions may be performed at temperatures between room temperature and the boiling temperature of solvents and mixtures. The final purification of friedelin is carried out by crystallisation.

The major novelty of these processes consists in the sequence and simplicity of the various mechanical, chemical and physical steps.

The initial step of the four methods of this invention consists in a milling operation of cork smoker wash solids which gives particles, with an equivalent diameter in the range of 250 µm - 5 cm.

In method 1, the second step consists in the treatment of cork smoker wash solids with an alkaline salt solution, being sodium hydroxide and sodium carbonate the preferred salts, followed by a third step of extraction with an aliphatic hydrocarbon, a mixture of aliphatic hydrocarbons or any oil fraction, in which the major components are the aliphatic C5 - C8 hydrocarbons at temperatures between room temperature and the boiling temperature of solvents, or mixtures of solvents. By distillation of the extracts obtained in the third step a fraction rich in friedelin is obtained, which is referred to as raw friedelin. Alternatively, raw friedelin may be obtained by adding a ketone or a low molecular weight alcohol to the extracts and by distilling the solvents. Raw friedelin is, then, submitted to a final purification procedure.

Method 2 is characterized by the same three steps of method 1. However, it additionally contains a fourth step whose purpose is to increase the friedelin content in the fraction referred to as raw friedelin. This fourth step consists in an extraction with an aliphatic hydrocarbon/aromatic hydrocarbon mixture, the heptane/toluene mixture being preferable, or with an aromatic hydrocarbon, toluene being preferable, or, in alternative, a fifth step, consisting in an extraction with an aromatic hydrocarbon if the fourth extraction is an extraction with aliphatic hydrocarbon/aromatic hydrocarbon. Aromatic hydrocarbon may be toluene or xylene, though toluene is preferred. In this method raw friedelin is obtained by adding a ketone or a low molecular weight alcohol to the extracts of aliphatic hydrocarbon, aliphatic hydrocarbon/aromatic hydrocarbon or aromatic hydrocarbon and by distilling the solvents. Raw friedelin may also be obtained merely by solvent distillation without the addition of a ketone or alcohol. Raw friedelin is then submitted to a final purification procedure.

The second step of method 3 consists in the extraction with an aliphatic hydrocarbon, mixture of aliphatic hydrocarbons or any oil fraction, in which the major components are aliphatic C5 - C8 hydrocarbons. The third step consists in an extraction, with a mixture of aliphatic/aromatic hydrocarbon. This method may or may not have a fourth step consisting in an extraction with an aromatic hydrocarbon. This step may be preferable, when one tries to obtain friedelin with a high purity degree, by simplifying the final purification step. Raw friedelin is obtained by adding a low molecular weight ketone or an alcohol to the extracts of aliphatic hydrocarbon/aromatic hydrocarbon and aromatic hydrocarbon and by distilling the solvents. Alternatively, raw friedelin may be obtained merely by solvent distillation, without the addition of a ketone or alcohol. Raw friedelin is then submitted to a final purification procedure.

The second step of method 4 consists in the extraction with a low molecular weight ketone, acetone being preferable. The third step consists in an extraction with an aliphatic hydrocarbon, the fourth step consists in an extraction with an aliphatic hydrocarbon/aromatic hydrocarbon mixture and the fifth step consists in an extraction with an aromatic hydrocarbon. Raw friedelin is obtained from the extracts with aliphatic hydrocarbon/aromatic hydrocarbon and with aromatic hydrocarbon produced in the fourth and fifth steps, through solvent distillation. Alternatively, raw friedelin may be obtained by adding a low molecular weight ketone or an alcohol to the extracts of aliphatic hydrocarbon/aromatic hydrocarbon and aromatic hydrocarbon and by distilling the solvents. Raw friedelin is then submitted to a final purification procedure.

### Friedelin Purification

Alternative methods for friedelin purification consist in
a) extraction with a low molecular weight alcohol followed by an extraction with ethyl acetate and recrystallisation of the residue in dichloromethane-ethyl acetate or toluene-ethyl acetate system.
b) extraction with a low molecular weight alcohol, treatment with an aqueous solution of an alkaline salt, or an aqueous solution of a low molecular weight alcohol and an alkaline salt, drying of the residue and pulverization of resulting material. This step is followed by an extraction with ethyl acetate, dichloromethane or chloroform. An additional extraction with toluene may be carried out. The final step consists in a recrystallisation in dichloromethane-ethyl acetate or toluene-ethyl acetate system.
c) treatment with an alkaline salt dissolved in an aqueous solution, or in a water and low molecular weight alcohol solution, drying of the residue and pulverization of the material. This step is followed by an extraction with chloroform or, alternatively dichloromethane, followed by an extraction with ethyl acetate and the recrystallisation in the dichloromethane-ethyl acetate or toluene-ethyl acetate system.
d) treatment with an alkaline salt dissolved in an aqueous solution or in a water and low molecular weight alcohol solution, drying of the residue and pulverization of the material. This step is followed by extractions with dichloromethane, ethyl acetate and isopropanol and the recrystallisation in the dichloromethane-ethyl acetate or toluene-ethyl acetate system.

The preferable alcohols are methanol, ethanol and isopropanol. The preferred alkaline salt is sodium hydroxide or sodium carbonate. These methods are illustrated by the following examples which are meant only to be illustrative, without limiting the scope of the invention.

### Example 1

**a)** Raw *Friedelin Production*
   1. Cork smoker wash solids were milled into pieces of 250 µm - 5 cm equivalent diameter and placed in a percolation column.
   2. 5 l of 2% sodium hydroxide aqueous solution were added to 2,5 Kg of fragmented material, and left in contact with the material to be extracted for 15 minutes. The column was discharged and water was added to the top of the column until the percolated solution showed a light colour.
   3. 5 I heptane were added and were left in the column for 1 hour, the column being then discharged.
   4. The material was then extracted with 17,5 I heptane at a temperature between 65°-70°C.
   5. The heptane extract obtained in step 4 was concentrated and a raw friedelin precipitate (about 143 g) was obtained. The friedelin content in this fraction varies between 50%-60%.
**b)** *Friedelin Purification*
   1. 250 ml methanol were added to 100 g raw friedelin. This solution was, heated to the boiling point and then filtered hot. This step was repeated.
   2. 300 ml of 20% sodium hydroxide solution and 700 ml ethanol were added to the residue treated by methanol. The mixture was heated at reflux for one hour, and then filtered.
   3. 100 ml ethyl acetate were added to the residue, stirred for half an hour and filtered.
   4. Toluene was added to the residue. The mixture was slightly heated and filtered after cooling the solution. In the filter a small amount of black material was separated. The solvent was distilled and ethyl acetate was added. After cooling friedelin crystals were obtained and filtered.
   5. The crystals were submitted to a final purification by recrystallisation in the dichloromethane-ethyl acetate system.

Friedelin with an 100% purity degree (HPLC) was obtained the global yield of the extraction was 1%.

### Example 2

**a)** Raw *friedelin production*
   1. Cork smoker wash solids were milled into pieces of 250 µm - 5 cm equivalent diameter and placed in a percolation column.
   2. 100 g of the fragmented material were treated with 400 ml of 5% sodium carbonate solution at 80°-90°C, for 5-10 minutes. A black suspension emulsion was formed and cooled to room temperature. After being acidified to pH 3-4, a solid deposit of phenolic nature was formed. The solid deposit was removed by filtration.
   3. The solid material contained in the column, not removed by the alkaline solution, was treated with 300 ml heptane at 65°-70°C. The heptane extract was cooled and a precipitate was obtained and separated from the solution by filtration.
      The remaining solution was evaporated until 1/5 of its volume and an equal volume of hot ethanol was added to the concentrated heptane solution. A precipitate was obtained.
   4. The remaining material was extracted with 100 ml boiling toluene. 12-15 g of black material of the same nature as the phenolic matrix material removed by sodium carbonate remained in the percolation column.
      The toluene solution was treated in the same way as the heptane solution.
   5. The precipitates obtained in step 4 were heated to the boiling point in 200 ml heptane. The solvent was decanted and concentrated, until 1/5 of its volume. An equal volume of ethanol was added. 4-5 g of a precipitate was obtained.
**b)** *Friedelin Purification*
   The precipitates resulting from steps 3 and 5 obtained from heptane and toluene/heptanes extracts were mixed together, boiled in ethanol and finally decanted. The residue was boiled with ethyl acetate and, after cooling, it was filtered over a Büchner's funnel. The friedelin crystals were submitted to a final purification by recrystallization in the dichloromethane-ethyl acetate system. The global yield of the extraction was 5%. Friedelin with a purity degree higher than 87% was obtained.

### Example 3

**a)** *Raw friedelin production*
   1. Cork smoker wash solids were milled into pieces of 250 µm - 5 cm equivalent diameter.
   2. 1 Kg cork smoker wash solids were placed in a column and 1,5 I heptane was added. The solid was left 30 minutes in contact with the solvent at a temperature of 65°-70°C.
   3. The percolation of the column was carried out during 30 minutes. 4. 1,5 I heptane at 65°-70°C were added to the column with the material already extracted by heptane and steps 2 and 3 were repeated.
   5. 1,5 l of a toluene/heptane mixture 1:1 was added to the column containing the material previously ectracted with heptane. The solvent was left in contact with the solid during 30 minutes at a temperature of 65°-70°C.
   6. The percolation of the column was carried out during 30 minutes.
   7. l I toluene/heptane mixture was then added and steps 5 and 6 were repeated.
   8. The toluene/heptane extracts obtained in steps 6 and 7 were mixed and concentrated until 1/6 of its volume. 1,5 l acetone was added, and left to rest overnight. About 130 g of a raw friedelin precipitate was formed. The friedelin content of this precipitate was about 40%.
**b)** *Friedelin Purification*
   1. 400 ml isopropanol were added to 50 g of raw friedelin, the mixture was heated to reflux and the extraction was repeated with 250 ml isopropanol.
   2. 50 ml of a 10% sodium hydroxide solution and 10 ml ethanol were added to the isopropanol extracted residue. The solution was heated to reflux for two hours. Most of the solvent was then removed by distillation and the residue was placed in an oven at 80°C for 24 hours to dry. The resulting material was pulverized.
   3. The pulverized product was extracted with 250 ml chloroform and filtered. 80 ml of 10% hydrochloric acid solution were added to the filtrate with stirring. The solution was dried with anhydrous sodium sulphate and it was then treated with 4 g of activated charcoal.
   4. A recrystallization in the system toluene/ethyl acetate (1:2) was perfomed. The yield of purified friedelin with a purity degree of 96% (HPLC) was 1,23%.

### Example 4

**a)** *Raw friedelin production*
   1. Cork smoker wash solids were fragmented into pieces of 250 µm - 5 cm equivalent diameter.
   2. 2,5 Kg cork smoker wash solids were placed in a column and 4 l acetone were added. The solids were left 30 minutes in contact with the solvent at room temperature.
   3. The percolation of the column was carried out during 30 minutes.
   4. 4 I heptane were added to the column containing the previously acetone extracted material and the solid was left 30 minutes in contact with the solvent at a temperature of 40°C.
   5. The percolation of the column was carried out at 55°C during 30 minutes.
   6. 4 I toluene/heptane mixture 7:3 were added to the column containing the previously heptane extracted material. The solid was left 30 minutes in contact with the solvent at a temperature of 75°C.
   7. The percolation of the column was carried out at 75°C during 30 minutes.
   8. The obtained percolation solution was cooled and then concentrated. Acetone was added in a quantity equal to the double of the concentrated solution and the resulting solution was cooled in an ice bath. A precipitate was obtained, washed with acetone and dried.
   9. 3 I toluene were added to the column containing the heptane/toluene extracted residue and the solid was left 30 minutes in contact with the solvent at a temperature of 80°C.
   10. The percolation of the column was carried out keeping the temperature of the column at 80°C. The mass of the solids in the column is about 1,2 Kg after drying.
   11. The resulting percolation solution was cooled and then concentrated. Acetone was added in a quantity equal to the double of the concentrated solution and the solution was cooled in an ice bath. The precipitate formed was washed with acetone and dried. This precipitate was added to the precipitate obtained in step 8.

   The total mass of the precipitates obtained in steps 8 and 10 is about 200 g. This precipitate is a friedelin rich fraction. This compound is present in this fraction in a content of about 60%.
**b)** *Friedelin purification*
   1. 10 ml of 10% NaOH aqueous solution and 50 ml ethanol were added to 50 g of raw friedelin and the mixture was heated to reflux for a period of 2 hours. Most of the solvent was removed by distillation and the resulting residue was placed in a oven at 80°C for a period of 24 hours. The resulting material was pulverized.
   2. Under stirring the residue was extracted with 600 ml chloroform for 1 hour and then filtered. 120 ml of a 7% hydrochloric acid solution were added to the extract solution under stirring. The solution was dried with anhydrous sodium sulphate and then was treated with 4 g of activated charcoal.
   3. Most of the solvent was eliminated by distillation under vacuum. 25 ml ethyl acetate were added and left in contact for a period of half an hour. The residue was filtered and washed with ethyl acetate.
   4. A recrystallization in toluene/ethyl acetate system was performed. Friedelin, with a purity degree of 87% (HPLC), corresponding to an extraction yield of extraction of 3% was obtained.

## Claims

1. Process for the extraction of friedelin, wherein the initial step is a fragmentation of cork smoker wash solids into particles with the equivalent diameter in the range of 250 µm- 5 cm.

2. Process for the extraction of friedelin according to claim 1, wherein the extraction of cork smoker wash solids is carried out by an alkaline salt solution at temperatures between room temperature and the boiling point of the solution.

3. Process according to claim 2, wherein the preferable alkaline salts are sodium hydroxide and sodium carbonate.

4. Process according to claims 2 and 3, wherein the residue resulting from the extraction with an alkaline salt solution is subsequently extracted with an aliphatic hydrocarbon, a mixture of aliphatic hydrocarbons or any oil fraction in which the major components are C5-C8 aliphatic hydrocarbons.

5. Process according to claim 4, wherein the extractions are carried out at temperatures between room temperature and the solvent, or the solvent mixture boiling point.

6. Process according to claims 4 and 5, wherein a friedelin rich in precipitate, referred to as raw friedelin, is obtained by distillation of the extracts.

7. Process according to claim 6, wherein friedelin may be obtained by precipitation and filtration of the precipitate obtained through the addition of a low molecular weight ketone or an alcohol to the extracts and by distilling off the solvents, the raw friedelin then being submitted to a final purification procedure.

8. Process according to claim 7, wherein the preferred ketone is acetone and the preferred alcohol is ethanol.

9. Process according to claims 1 to 5, wherein the extraction residue resulting from the steps described in claim 4 is extracted with a mixture of an aliphatic hydrocarbon/aromatic hydrocarbon, the heptane/toluene mixture being preferred or with an aromatic hydrocarbon toluene being preferable.

10. Process according to claim 9, wherein a fifth step is carried out, consisting in an extraction with an aromatic hydrocarbon if the fourth extraction consists in an extraction with an aliphatic hydrocarbon/aromatic hydrocarbon mixture, in which the aromatic hydrocarbon may be toluene or xilene, toluene being preferable.

11. Process according to claims 9 and 10, wherein the extractions are carried out at temperatures between room temperature and the solvent, or solvent mixture boiling point.

12. Process according to claims 9, 10 and 11, wherein friedelin is obtained by filtration of the precipitate obtained through the addition of a ketone or of a low molecular weight alcohol to the extracts of aliphatic hydrocarbon, aliphatic hydrocarbon/aromatic hydrocarbon, or aromatic hydrocarbon and the resulting raw friedelin being then submitted to a final purification procedure.

13. Process according to claim 12, wherein the preferred ketone is acetone and the preferred alcohol is ethanol.

14. Process according to claims 9, 10 and 11, wherein friedelin may be obtained merely by the solvent distillation without the addition of a ketone or alcohol and the precipitate thus obtained being then submitted to a final purification procedure.

15. Process of extraction according to claim 1, wherein a second step is carried out consisting in an extraction of cork smoker wash solids with an aliphatic hydrocarbon, a mixture of aliphatic hydrocarbons, or any oil fraction, in which the major components are C5-C8 aliphatic hydrocarbons.

16. Process of extraction according to claim 15, wherein a third step, consisting in an extraction with an aliphatic hydrocarbon/aromatic hydrocarbon mixture is carried out.

17. Process of extraction according to claim 16, wherein a fourth step, consisting in an extraction with an aromatic hydrocarbon is carried out.

18. Process according to claims 15, 16 and 17, wherein the extractions are carried out at temperatures between room temperature and the solvent, or the solvent mixture boiling point.

19. Process according to claims 16 and 17, wherein friedelin may be obtained by adding a low molecular weight ketone or an alcohol to the extracts of aliphatic hydrocarbon/aromatic hydrocarbon and aromatic hydrocarbon and then distilling the solvents and the precipitate thus obtained being then submitted to a final purification procedure.

20. Process according to claim 19, wherein the preferred ketone is acetone and the preferred alcohol is ethanol.

21. Process according to claims 16 and 17, wherein friedelin may be obtained merely by the solvent distillation without the addition of a ketone or alcohol, and the precipitate thus obtained being then submitted to a final purification procedure.

22. Process of extraction according to claim 1, wherein a second step consisting in an extraction of cork smoker wash solids with a low molecular weight ketone is carried out.

23. Process of extraction according to claim 22, wherein the preferred ketone is acetone.

24. Process of extraction according to claims 22 and 23, wherein a third step consisting in an extraction with an aliphatic hydrocarbon is carried out.

25. Process of extraction according to claims 22, 23 and 24, wherein a fourth step consisting in an extraction with an aliphatic hydrocarbon/aromatic hydrocarbon mixture is carried out.

26. Process of extraction according to claim 25, wherein a fifth step consisting in an extraction with an aromatic hydrocarbon is carried out.

27. Process of extraction according to claims 22 to 26, wherein the extractions are carried out at a temperature between room temperature and the solvent, or the solvent mixture boiling point.

28. Process of extraction according to claims 25 and 26, wherein a raw friedelin precipitate is obtained by solvent distillation of aliphatic hydrocarbon/aromatic hydrocarbon extracts and from aromatic hydrocarbon extracts, resulting from the fourth and fifth steps.

29. Process of extraction according to claims 25 and 26, wherein friedelin is obtained by adding a low molecular weight ketone or an alcohol to the aliphatic hydrocarbon/aromatic hydrocarbon and aromatic hydrocarbon extracts and by distilling the solvents and the raw friedelin precipitate thus obtained is then submitted to a final purification procedure.

30. Process according to claim 29, wherein the preferred ketone is acetone and the preferred alcohol is ethanol.

31. Process for final purification of friedelin, wherein raw friedelin is submitted to an extraction with a low molecular weight alcohol, followed by an extraction with ethyl acetate and a recrystallization in a dichloromethane-ethyl acetate or toluene-ethyl acetate system.

32. Process according to claim 31, wherein the preferred alcohols are methanol, ethanol or isopropanol.

33. Process for the final purification of friedelin, wherein an extraction with a low molecular weight alcohol, treatment with an alkaline salt dissolved in an aqueous solution, or in a water and low molecular weight alcohol solution, drying of the residue and pulverization of the material are carried out.

34. Process according to claim 33, wherein the preferred alkaline salt is sodium hydroxide, or sodium carbonate and the preferred alcohols are methanol, ethanol and isopropanol.

35. Process according to claims 33 and 34, wherein the pulverized material is extracted with ethyl acetate, dichloromethane or chloroform, optionally followed by an additional extraction with toluene.

36. Process according to claims 33, 34 and 35, wherein a recrystallization in a dichloromethane-ethyl acetate or toluene-ethyl acetate system is carried out, yielding a friedelin with a purity degree higher than 87%.

37. Process for final purification of friedelin, wherein a treatment with an alkaline salt dissolved in an aqueous solution, or in a water and low molecular weight alcohol solution, drying of the residue and pulverization of the material are carried out.

38. Process according to claim 37, wherein the preferred alkaline salt is sodium hydroxide, or sodium carbonate, and the preferred alcohols are methanol, ethanol and isopropanol.

39. Process according to claims 37 and 38, wherein the pulverized material is extracted with chloroform or in alternative with dichloromethane, followed by an extraction with ethyl acetate.

40. Process according to claims 37, 38 and 39 wherein a recrystallisation in the dichloromethane-ethyl acetate or toluene-ethyl acetate system is carried out, yielding friedelin with a purity degree higher than 87%.

41. Process for final purification of friedelin, wherein an hydrolysis with an alkaline salt dissolved in an aqueous solution or in water and a low molecular weight alcohol solution, drying of the residue and pulverization of the material are carried out.

42. Process according to claim 41, wherein the preferred alkaline salt is sodium hydroxide or sodium carbonate and the preferred alcohols are methanol, ethanol and isopropanol.

43. Process according to claims 41 and 42, wherein the pulverized material is extracted with dichloromethane, ethyl acetate and isopropanol.

44. Process according to claims 41, 42 and 43, wherein a recrystallization in a dichloromethane-ethyl acetate or toluene-ethyl acetate system is carried out yielding friedelin with a purity degree higher than 87%.
